Europäisches Patentamt

European Patent Office

Office européen des brevets

(11) Publication number: **0 514 810 A1**

## EUROPEAN PATENT APPLICATION

(21) Application number: **92108348.1**

(22) Date of filing: **18.05.92**

(51) Int. Cl.5: **A61B 17/32**, A61B 17/22

(30) Priority: **22.05.91 IT MI911415**

(43) Date of publication of application:
**25.11.92 Bulletin 92/48**

(84) Designated Contracting States:
**AT BE CH DE DK ES FR GB GR IT LI LU MC NL PT SE**

(71) Applicant: **La Rosa, Antonio**
**Via Toledina 2**
**I-27026 Garlasco,(Pavia)(IT)**

(72) Inventor: **La Rosa, Antonio**
**Via Toledina 2**
**I-27026 Garlasco,(Pavia)(IT)**

(74) Representative: **Gervasi, Gemma, Dr. et al**
**NOTARBARTOLO & GERVASI Srl 33, Viale Bianca Maria**
**I-20122 Milano(IT)**

(54) **Ultrasonic dissector and detacher for atherosclerotic plaques.**

(57) An ultrasonic dissector and detacher for atherosclerotic plaques allowing surgical removal in a totally correct manner of atherosclerotic plaques from the affected vessels is described. Said dissector and detacher comprises:
- an ultrasonic frequency oscillator (3),
- an ultrasonic probe (1) connected to said oscillator (3),
- a tool (6) connected mechanically to said probe (1),
- means (7) integral with said probe (1) to wet said tool (6) with a liquid.

fig. 1

EP 0 514 810 A1

## Field of the invention

The present invention refers to an ultrasonic surgical tool for dissection and detachment of atherosclerotic plaques.

Said tool is particularly useful in the field of vascular surgery because it fulfils the dual function of dissection and detachment of atherosclerotic plaques and allows their removal in an optimal manner.

## State of the art

As known, atherosclerosis is an ailment which affects the wall of arteries such as the aorta, coronaries, cerebral arteries and those of the limbs, especially the lower limbs, with the formation of focal and diffused thickening (plaques) of lipidic and fibrous material which can evolve occlusively and be complicated by ulcerative and thrombotic phenomena.

Atherosclerosis is certainly not a recent disease, being found even in arterial fragments of Egyptian mummies but it seems that its incidence has considerably increased in recent years because the number of arteries occlusions clinically evidenced and connected therewith has considerably increased if compared with the past.

Occlusive thrombotic phenomena cause a drastic reduction of the vessel patency which can lead to sometimes very serious consequences, in particular in small and medium diameter arteries; for these reasons the open-vessel surgery is used more and more to remove atherosclerotic plaques from the arteries involved and restore regular blood flow through the vessel.

## Technical problem

Removal of atherosclerotic plaques from artery walls is often performed in vascular surgery with the open vessel technique. After incision of the vessel with a lancet, the atherosclerotic plaque is detached manually from the underlying tissues by special dissectors and finally removed.

Conventional dissectors however have the disadvantage of not ensuring perfect detachment of the atherosclerotic plaque from the vessel wall, leaving a rather irregular surface with fragments and small edges of plaque which, if not removed, can be responsible for post-operative microembolic complications.

## Outline of the invention

It has now been found possible to surgically remove in a more effective manner atherosclerotic plaques from the affected vessels if the ultrasonic dissector and detacher which is the object of the present invention is used; said tool comprises:

- an ultrasonic frequency oscillator,
- an ultrasonic probe connected to the oscillator,
- a tool connected mechanically to said probe,
- means integral with said probe to wet said tool with a liquid.

## List of drawings

The invention will be better described with reference to a nonlimiting example of an embodiment illustrated in the annexed drawings where;

FIG. 1 shows a block diagram of a possible embodiment of the invention,

FIG. 2 shows one of the possible embodiments of the tool 6,

FIGS. 3 to 6 show the active zone of tools 6 different from those illustrated in the above FIGS., and

FIGS. 7 and 8 show front view and side view of tools 6 different from those illustrated in the above FIGS.

## Detailed description

There are already known and used in the medical field (dentistry, organ surgery, ablation of calculi) ultrasonic equipment which causes fragmentation, destruction or sectioning of the target (dental plaque; tissue or calculi) and cannot therefore be used in the field of vascular surgery where it is very important to leave the lumen of the vessel free of fragments or roughness.

Object of the present invention is a new and original device which allows the use of ultrasounds for removal of atherosclerotic plaques.

To remove an atherosclerotic plaque through the above device, the vessel to be treated is open, the plane of cleavage of the plaque (i.e. the plane along which the plaque leaves readily the vessel wall without damaging said wall) is identified, the most suitable tool is mounted on the probe (or a probe already fitted with said tool is used) the end portion (called below "active zone") of said tool is inserted in the plane of cleavage and made to proceed in the vessel lumen. The ultrasonic energy delivered by the probe to the tool causes on the active zone wetted by a sterile liquid a cavitation effect (with formation of steam bubbles which does on the surrounding tissues a pressure of 2-3 atmospheres) which, together with the mechanical action of the active zone which oscillates at ultrasonic frequency, allows a regular and uniform detachment of the plaque from the vessel wall according to the initial plane of cleavage.

The atherosclerotic plaque detached along a uniform plane without damaging the vessel wall is completely removed without leaving in the lumen of the vessel small rough spots, fragments or residue of any type.

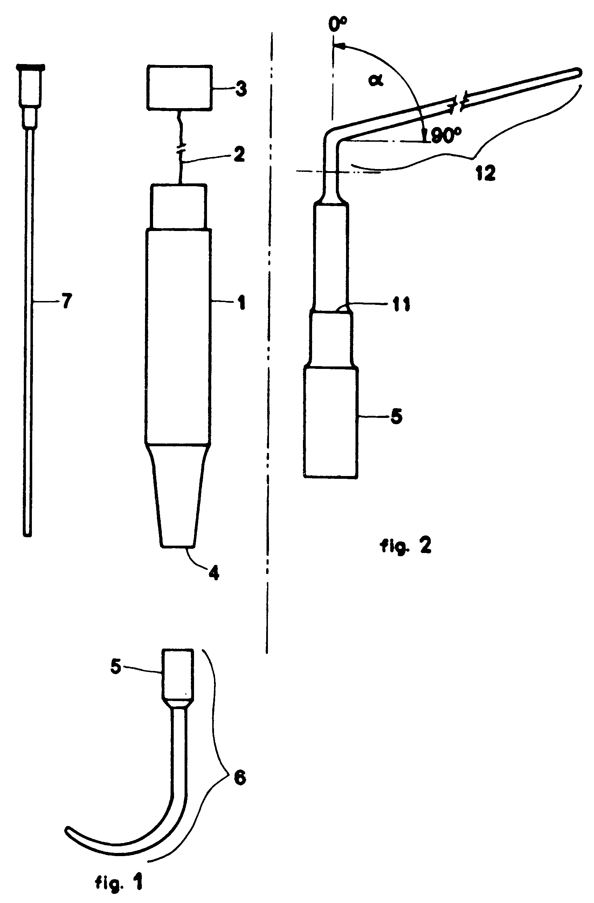

FIG. 1 shows a block diagram of a possible embodiment of an ultrasonic dissector and detacher relized according to the present invention; reference number 1 indicates the ultrasonic probe (preferably a piezoelectric crystal probe) fed through the cable 2 from the ultrasonic frequency oscillator 3 whose output signal in a preferred embodiment has a frequency preferably between 20kHz and 29kHz.

To limit the heat developed by the probe 1 during its working, said probe is cooled by water or some other sterile fluid made to run inside the probe 1 through a connecting cable 2 or by oil (or some other equivalent fluid) enclosed in a sealed cooling circuit.

At the other end 4 of the probe 1 is connected by fastening means 5 one of the interchangeable tools 6 better described in the subsequent figures; in a preferred embodiment the tools 6 are realized in steel for the surgical tools but, without going beyond the scope of the invention, said tools can be made of any material (e.g. titanium or its alloys) suitable for the purpose.

In accordance with another possible embodiment (not shown illustrated in the figures) of a dissector and detacher realized according to the present invention the tool 6 is connected rigidly to the probe 1. The probe 1 and the relevant tool 6 constitute thereby an enbloc tool to be discarted after use.

The use of such a disposable tool can be advantageous because it avoids unnecessary sterilization costs of used tools 6 and the risks related to a reuse of said tools 6 even if sterilized correctly, contributing to more effective prophylaxis against diseases transmissible through the blood such as AIDS and type B or type not-A not-B viral hepatitis.

In figures, reference 7 indicates a tube (of the single-use type or reusable because made of sterilizable material) which, made integral with the probe 1 by coupling means not explicitly indicated in the figures, allows wetting of the tool 6 with a sterile liquid (preferable a physiological solution) coming from a tank (not shown in the figures) connected to the tube 7 in a known manner.

In accordance with another embodiment of the invention (not shown in the figures) the tube which allows wetting of the tool 6 with said sterile liquid is connected (by clipping in or some other known means) to at least one channel made in the body of the tool 6.

In accordance with another possible embodiment of the invention (also not illustrated in the figures) the tool 6 is wetted with the above sterile liquid through a catheter which covers the tool body leaving free only the active zone. The catheter is connected to the tank containing the sterile liquid by a tube 7 or at least one channel made in the body of the tool 6.

FIG. 2 shows schematically one of the tools 6, which are mutually interchangeable, which (according to the embodiment shown in FIG. 1) can be mounted on the probe 1; said tool (having a form different from that of the tool 6 shown in FIG. 1) comprises fastening means 5 which allow the assembling of the tool 6 to the probe 1 in a reversible manner (e.g. by a screw or bayonet coupling), the active zone 12 (delimited in the figure by a broken line) and an intermediate zone 11 which connects the active zone 12 to the reversible fastening means 5.

In a disposable tool the means 5 are made up of the fastening means designed to connect in an irreversible manner the intermediate zone 11 with the end of the probe 1.

Preferably the active zone 12 has a length between 5mm and 70mm, width between 1.5mm and 4mm and a thickness between 0.2mm and 2mm and is inclined to the axis of the intermediate zone 11 at an angle $\alpha$ between 0° and 90°.

To allow ready fastening of the tool 6 to the probe 1, it is opportune that the dimensions of the means 5 are greater than those of the active zone 12 to which they are connected through the intermediate zone 11 which, in the tool described in FIG. 2, is formed of cylindrical overlapping bodies having a progressively diminishing diameter but which can be made up of one or more truncated cone bodies or have the form considered from time to time most advantageous for the specific application.

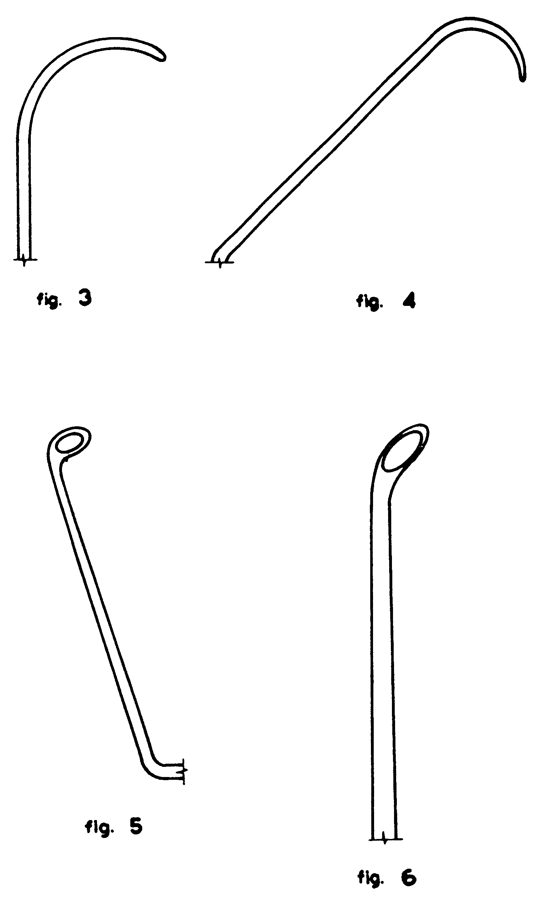

FIGS. 3 to 8 show the active zone 12 of some tools 6 made in accordance with the present invention; the fastening means 5 and the intermediate zone 11 are omitted for the sake of simplicity of graphic representation.

In said figures there can be noted the following possible characteristic elements of the active zone 12:

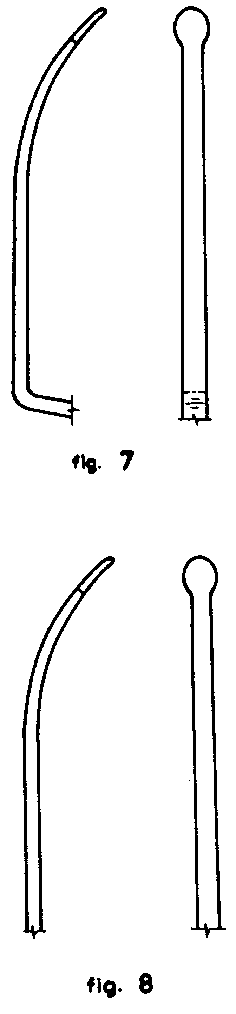

- at least in the terminal portion, it has a curved profile (side view of FIGS. 7 and 8) or a profile represented by a segment of a circle (FIGS. 1, 3 and 4);
- the terminal portion is spatula shaped (front view of FIGS. 7 and 8),
- it has at the end a closed eyelet (FIGS. 5 and 6) inclined to the axis of the active zone 12 by an angle between 0° and 90° and preferably between 35° and 45°, the length of said eyelet being between 3cm and 50cm,
- before its inclined zone a first short section aligned with the axis of the intermediate zone 11 is provided (FIGS. 2, 4, 5, 7).

Without going beyond the scope of the invention it is possible for one skilled in the art to combine two or more of the above mentioned characteristic elements together and with those (dimensions, etc.) shown in the description of FIG. 2 to realize tools 6 having a shape different from those illustrated by way of example in the FIGS. and give to the ultrasonic dissector and detacher which is the object of the present description all the modifications suggested by normal experience and the natural evolution of the specific technique.

**Claims**

1. Ultrasonic dissector and detacher for atherosclerotic plaques characterized in that it comprises:
   - an ultrasonic frequency oscillator (3),
   - an ultrasonic probe (1) connected to said oscillator (3),
   - a tool (6) connected mechanically to said probe (1), and
   - means (7) integral with said probe (1) to wet said tool (6) with a liquid.

2. Ultrasonic dissector and detacher as in claim 1 characterized in that said tool (6) is connected to said probe (1) in a reversible manner.

3. Ultrasonic dissector and detacher as in claim 1 characterized in that said tool (6) is connected to said probe (1) in a rigid manner.

4. Ultrasonic dissector and detacher as in claim 1 characterized in that the output signal of said oscillator (3) has a frequency between 20kHz and 29kHz.

5. Ultrasonic dissector and detacher as in claim 1 characterized in that said wetting means (7) consists of a tube applied to the outside of said probe (1) connected to a tank containing said liquid.

6. Ultrasonic dissector and detacher as in claim 1 characterized in that said wetting means (7) consist of a tube connected to a tank containing said liquid connected to at least one channel made in the body of said tool (6).

7. Ultrasonic dissector and detacher as in claim 1 characterized in that said wetting means (7) consist of a catheter fitted over the body of said tool (6) and connected to a tank containing said liquid.

8. Ultrasonic dissector and detacher as in claim 1 characterized in that said tool (6) comprises fastening means (5) to said probe (1) and an active zone (12) connected together by an intermediate zone (11).

9. Ultrasonic dissector and detacher as in claim 8 characterized in that said active zone (12) has a length between 5mm and 70mm, a width between 1.5mm and 4mm and a thickness between 0.2mm and 2mm and is inclined to the axis of said intermediate zone (11) by an angle ($\alpha$) between 0° and 90°.

10. Ultrasonic dissector and detacher as in claim 8 characterized in that said active zone (12) has a straight line trend.

11. Ultrasonic dissector and detacher as in claim 8 characterized in that at least the end portion of said active zone (12) has a curved profile.

12. Ultrasonic dissector and detacher as in claim 8 characterized in that the terminal portion of said active zone (12) is spatula-shaped.

13. Ultrasonic dissector and detacher as in claim 8 characterized in that the terminal portion of said active zone (12) has a closed eyelet the length of which is between 3cm and 50cm.

14. Ultrasonic dissector and detacher as in claim 1 characterized in that it also comprises a circuit for cooling said probe (1).

fig. 2

fig. 1

fig. 3

fig. 4

fig. 5

fig. 6

fig. 7

fig. 8

European Patent
Office

**EUROPEAN SEARCH REPORT**

Application Number

EP 92 10 8348

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | US-A-3 433 226 (BOYD)<br>* Column 3, line 74 - column 4, line 12 * | 1,2,4-6,8,14 | A 61 B 17/32<br>A 61 B 17/22 |
| X | GB-A-2 176 110 (NIPPON INFRARED INDUSTRIES CO., LTD)<br>* Page 2, lines 11-15 * | 1,3-6,14 | |
| X<br>A | AT-A- 340 572 (REDTENBACHER et al.)<br>* Page 2, lines 36-49 * | 1,8-12,14<br>13 | |
| X<br><br>A | US-A-4 962 755 (KING et al.)<br>* Column 4, lines 34-40,48-57 * | 1,2,4-8,10-12,14<br>9 | |
| A | EP-A-0 238 667 (SUMITOMO)<br>* Page 8, lines 4-7; figure 2 * | 1,8,13 | |
| A | US-A-3 086 288 (BALAMUTH et al.)<br>* Column 6, lines 10-13; figure 1 * | 1,14 | TECHNICAL FIELDS SEARCHED (Int. Cl.5)<br><br>A 61 B<br>A 61 F |

The present search report has been drawn up for all claims

| Place of search | Date of completion of the search | Examiner |
|---|---|---|
| THE HAGUE | 13-08-1992 | GLAS J. |